# EUROPÄISCHE PATENTANMELDUNG

(11) **EP 1 772 140 A1**
(43) Veröffentlichungstag der Anmeldung: **11.04.2007**
(21) Anmeldenummer: 06121960.6
(22) Anmeldetag: 09.10.2006
(51) Int. Cl.: A61K 8/97, A61Q 19/00

(54) **Kosmetische Formulierungen zur Verbesserung der Hautbarrierefunktion**

(30) Priorität: 10.10.2005 DE 102005048779
(71) Anmelder: Beiersdorf AG, 20253 Hamburg (DE)
(72) Erfinder: Neufang, Gitta, 20251, Hamburg (DE); Kolbe, Ludger, 21255, Dohren (DE); Mummert, Christopher, 29553, Bienenbüttel (DE); Raschke, Thomas, 25421, Pinneberg (DE); Eckert, Julia, 20257, Hamburg (DE); Immeyer, Jeannine, 21272, Egestorf-Sahrendorf (DE)

(57) **Zusammenfassung**

Verwendung von kosmetischen Formulierungen mit einem Gehalt an Silybin und/oder Silydianin und/oder Silychristin oder Silymarin zur Erhöhung bzw. Wiederherstellung der Barriereeigenschaften der Haut.

## Beschreibung

Die vorliegende Erfindung betrifft die Verwendung kosmetischer Zubereitungen mit einem kosmetisch wirksamen Gehalt an Silymarin zur Verbesserung zur Verbesserung der Hautbarrierefunktion.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen. Schon vor Jahrtausenden wurde Kosmetik vom Menschen zu diesem Zweck angewandt. Man färbte Lippen und Gesicht, salbte sich mit wertvollen Ölen und badete in duftendem Wasser.

Während die Reinigungswirkung eines Shampoos, die Kämmbarkeitsverbesserung einer Haarspülung oder die Kräuselung des Haares durch eine Dauerwelle einfach und objektiv feststellbar sind, sind andere Wirkungen und Eigenschaften kosmetischer Produkte praktisch nicht messbar oder nur sehr individuell spürbar. Hierzu zählen zum Beispiel ein bestimmtes (belebendes, weiches, geschmeidiges, glattes etc.) Hautgefühl oder die Weichheit und Geschmeidigkeit der Haut nach der Anwendung eines Kosmetikums sowie die Fülle und Sprungkraft der Haare und dergleichen mehr.

Eine für den Verbraucher sehr wesentliche, dabei aber nur schwer quantitativ meßbare Eigenschaft kosmetischer Produkte ist ein bestimmtes belebendes und/oder glattes Hautgefühl sowie die Geschmeidigkeit der Haut nach seiner Anwendung.

Aufgabe der vorliegenden Erfindung war es, hautpflegende Zubereitungen und Zubereitungen zur Pflege der auf natürliche Weise gealterten Haut zur Verfügung zu stellen, wobei die Zubereitungen das Körperäußere pflegen und verschönern sollten.

"Silymarin" ist die technische Bezeichnung eines Pflanzenextrakts aus den Früchten der Mariendistel (Silybum Marianum), welcher die drei Substanzen Silybin, Silydianin und Silychristin in unterschiedlichen Konzentrationen enthalten kann. Silybin, Silydianin und Silychristin sind durch die folgenden Strukturformeln gekennzeichnet:

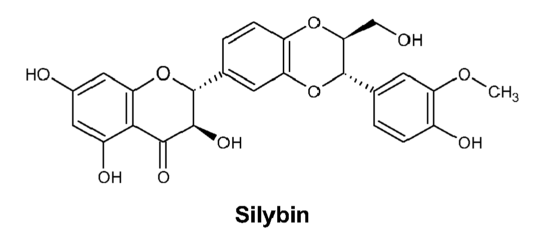

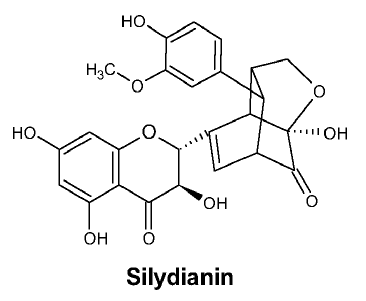

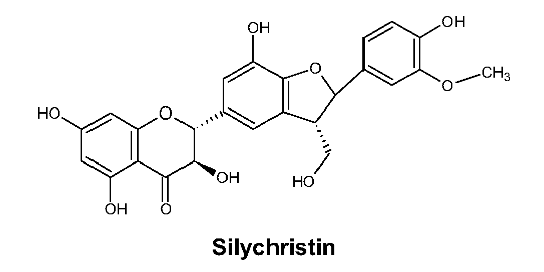

Die Verwendung von Silymarin in Zubereitungen zur Behandlung der Haut ist an sich bekannt.

So beschreibt beispielsweise die EP 0 300 282 topische pharmazeutische oder kosmetische Zubereitungen, welche Silymarin enthalten. Allerdings ist diese Schrift zu allgemein, als dass sie den Weg zur vorliegenden Erfindung hätte weisen können.

Die EP 0 552 439 beschreibt pharmazeutische Präparate enthaltend Silymarin zur topischen Behandlung von Psoriasis und atopischer Dermatitis. Die Behandlung von Psoriasis und atopischer Dermatitis sind nicht Gegenstand der vorliegenden Patentanmeldung.

Die US 5 972 993 offenbart eine Methode zur Behandlung von Rosacea oder und/oder Erröten, die darin besteht, eine Zubereitung, welche bestimmte Antioxidantien enthält, auf die Haut aufzutragen, wobei als Antioxidans - neben anderen möglichen Substanzen - auch Silymarin genannt wird. Die Behandlung von Rosacea oder und/oder Erröten sind nicht Gegenstand der vorliegenden Patentanmeldung.

Die WO 99/63982 (University of Medicine & Dentistry of New Jersey) beschreibt eine Methode zur Wundheilung, die darin besteht, eine Zubereitung, welche bestimmte Inhibitoren enthält, mit der Wunde in Kontakt zu bringen, wobei als Inhibitor - neben anderen möglichen Substanzen - auch Silymarin genannt wird. Diese Methode eigne sich auch zur Behandlung von Windeldermatitis. Die Behandlung von Windeldermatitis ist nicht Gegenstand der vorliegenden Patentanmeldung.

Die US 5 804 168 (Murad, Howard) beschreibt pharmazeutische Zubereitungen enthaltend mindestens ein Antioxidans, mindestens eine antientzündlich wirkende Komponente und mindestens eine Substanz, die das Immunsystem stärkt, wobei als Antioxidans - neben anderen möglichen Substanzen - auch Silymarin genannt wird. Die beschriebenen Zubereitungen sollen vor Schäden schützen bzw. Schäden behandeln, die dadurch entstehen können, dass die Haut schädigender Lichteinwirkung ausgesetzt wird. Dazu werden die Zubereitungen bevorzugt oral verabreicht (Spalte 4, ab Zeile 45). Zwar werden auch topische Zubereitungen genannt, allerdings konnte diese Schrift nicht den Weg zur vorliegenden Erfindung weisen.

Die US 2004/0087516-A1 offenbart eine Methode zur Behandlung von peripheralen neuralen und vaskularen Beschwerden, die darin besteht, eine Zubereitung, welche eine therapeutisch wirksame Menge bestimmter Flavonoide enthält, auf die Haut aufzutragen, wobei als Flavonoide - neben anderen möglichen Substanzen - auch Silymarin genannt wird.

Es war vielmehr überraschend und darin liegt die Lösung dieser Aufgaben, dass
die Verwendung von kosmetischen Formulierungen mit einem Gehalt an Silybin und/oder Silydianin und/oder Silychristin oder Silymarin zur Erhöhung bzw. Wiederherstellung der Barriereeigenschaften der Haut den Nachteilen des Standes der Technik abhelfen würden.

Die Formulierungen im Sinne der vorliegenden Erfindung sind in jeglicher Hinsicht überaus befriedigende Präparate, die sich durch eine langanhaltende Pflegewirkung auszeichnen. Die erfindungsgemäße Verwendung der kosmetischen Zubereitungen mit einem kosmetisch wirksamen Gehalt an Silymarin - z. B. in Form der genannten Beispiele - führt in überraschender Weise zu einer erheblichen Verbesserung des Aussehens der Haut, insbesondere werden durch die erfindungsgemäße Verwendung
■ die Barriereeigenschaften der Haut erhalten oder wiederhergestellt,
■ besser der Hautaustrocknung entgegengewirkt sowie
■ die Haut besser vor Umwelteinflüssen geschützt bzw. ihre Belastbarkeit gegenüber Umweltreizen erhöht.

Ferner wird durch die erfindungsgemäße Verwendung die mechanische Stabiliät der Haut gestärkt und ihre Spannkraft und Vitalität erhöht. Es war ferner erstaunlich, dass sich die Haut nach der erfindungsgemäßen Verwendung straffer anfühlt.

Es war insbesondere überraschend, dass durch die erfindungsgemäße Verwendung auch die Barriereeigenschaften der Haut von Kindern und Jugendlichen deutlich verbessert werden. Die Haut von Kindern und Jugendlichen besitzt im Vergleich zur Haut erwachsener Menschen (definitionsgemäß ist die Grenze zwischen Jugend- und Erwachsenenstatus der Abschluss der Pubertät) eine schwächere Hautbarriere. Dies hat zur Folge, dass Kinder- und/oder Jugendhaut eine vergleichsweise hohe Anfälligkeit für Schädigungen und Verletzungen hat. Ferner können aufgrund der schlechteren Barrierefunktion leichter reizende Noxen eindringen, weshalb die Haut von Kindern und Jugendlichen üblicherweise sensibler auf die Einwirkung von externen Noxen reagiert. Durch eine Anwendung von Silymarin im Sinne der vorliegenden Erfindung wird überraschenderweise die Hautbarrierefunktion bei Kindern und Jugendlichen gestärkt - die Haut ist daher besser vor schädlichen Umwelteinflüssen geschützt.

Die Barrierewirkung der Haut kann über eine Bestimmung der Reduktion des transepidermalen Wasserverlustes (TEWL - transepidermal water loss) quantifiziert werden. Beim TEWL handelt es sich um die Abdunstung von Wasser aus dem Körperinneren ohne die Einbeziehung des Wasserverlustes beim Schwitzen. Der TEWL kann z. B. in vivo bestimmt werden. Das Verfahren wird im folgenden stichpunktartig erläutert:
- Die Messung des TEWL erfolgt über der Haut des Probanden.
- Gemessen wird die verdunstende Menge Wasser über einem definierten Messareal.
- Anhand der Verringerung des verdunstenden Wassers über einem behandelten Hautareal im Vergleich zu einem unbehandelten Hautareal kann die Verbesserung der Hautbarriere errechnet werden.
- Das Messprinzip beruht auf der Veränderung des elektrischen Widerstands an der verwendeten Messsonde anhand der verdunstenden Wassermenge.

Mit "Silymarin" werden im Sinne der vorliegenden Erfindung Stoffgemische bezeichnet, welche (mindestens) die drei Substanzen Silybin, Silydianin und Silychristin in unterschiedlichen Konzentrationen enthalten. Dabei ist es unwesentlich, in welchen Verhältnissen diese Stoffe zueinander vorliegen und ob noch weitere Substanzen in dem Gemisch vorhanden sind. Vorteilhaft im Sinne der vorliegenden Erfindung sind z. B. die unter den Handelsbezeichnungen Silymarin und Silymarin S von Indena erhältlichen Silymarine.

Vorteilhaft ist auch das in der EP 300 282 B1 offenbarte Gemisch aus Silybin, Silydianin und Silychristin mit natürlichen oder synthetischen Phospholipiden und Bindemitteln. Eine vorteilhafte Ausführungsform ist beispielsweise unter der Handelsbezeichnung Siliphos bei der Firma Indena erhältlich.

Die Zubereitungen im Sinne der vorliegenden Erfindung enthalten vorteilhaft z. B. 0,001 Gew.-% bis 10 Gew.-%, vorzugsweise 0,01 bis 5 Gew.-%, insbesondere 0,02 bis 0,5 Gew.-% der erfindungsgemäßen Wirkstoffe, jeweils bezogen auf das Gesamtgewicht der Zubereitungen. Die erfindungsgemäßen Wirkstoffe können dabei einzeln oder als Mischungen miteinander vorliegen und rein oder in Form von Pflanzenextrakten oder anderen Gemischen mit weiteren Substanzen eingesetzt werden.

Die Zubereitungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Formulierungen können vorzugsweise auch Mikroemulsionen, Stifte, Schäume (sog. Mousse), Feststoff-Emulsionen (d. h. Emulsionen, welche durch Feststoffe stabilisiert sind, z. B. Pickering-Emulsionen), sprühbare Emulsionen oder Hydrodispersionen sein. Besonders bevorzugt bestehen die erfindungsgemäßen Formulierungen im wesentlichen aus einer Öl- und einer Wasserphase, wobei sie geringe Anteile an multiplen Tröpfchen herstellungsbedingt enthalten können.

Die kosmetischen Formulierungen im Sinne der vorliegenden Erfindung können wie üblich zusammengesetzt sein und insbesondere zur Behandlung und der Pflege der Haut und/oder der Haare sowie als Schminkprodukt in der dekorativen Kosmetik dienen. Entsprechend können die Formulierungen im Sinne der vorliegenden Erfindung -je nach ihrem Aufbau - beispielsweise verwendet werden als Hautschutzcrème, Reinigungsmilch, Sonnenschutzlotion, Nährcrème, Tages- oder Nachtcrème usw. Bevorzugt sind insbesondere solche kosmetischen Formulierungen, die in Form von O/W-Emulsionen vorliegen.

Zur Anwendung werden die kosmetischen Formulierungen im Sinne der vorliegenden Erfindung in der für Kosmetika üblichen Weise auf die Haut und/oder die Haare in ausreichender Menge aufgebracht.

Die kosmetischen Formulierungen im Sinne der vorliegenden Erfindung können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel und/oder Silikonderivate sowie Moisturizer.

Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure und/oder ein fucosereiches Polysaccharid, welches in den Chemical Abstracts unter der Registraturnummer 178463-23-5 abgelegt und z. B. unter der Bezeichnung Fucogel® 1000 von der Gesellschaft SOLABIA S.A. erhältlich ist.

Die jeweils einzusetzenden Mengen an kosmetischen Hilfs- und Trägerstoffen und Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische Anwendungen geeigneten oder gebräuchlichen Antioxidantien verwendet werden.

Vorteilhaft werden die Antioxidantien gewählt aus der Gruppe bestehend aus Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Rutinsäure und deren Derivate, alpha-Glycosylrutin und Butylhydroxytoluol.

Die Menge der vorgenannten Antioxidantien (eine oder mehrere Verbindungen) in den Emulsionen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Um die Löslichkeit der an sich schwerlöslichen Silymarine zu verbessern, kann die Lipidphase bzw. Ölphase der erfindungsgemäßen Zubereitungen vorteilhaft gewählt werden aus folgendern Substanzgruppen:
- Öle mit lösungsvermittelnden Eigenschaften, wie Rizinusöl und Ethylhexylmethoxycinnamat
- synthetische Öle mit hohem Gehalt an Esterbindungen, bevorzugt aus der Gruppe AIkylbenzoate, Alkyllaktate, Alkylmalate, Alkyltartrate, Dibutyladipate, Pentaerythrityl Tetraisostearate
- Glykolester, bevorzugt aus der Gruppe der Propylene Glykol Isostearate, Propylene Glycol Monolaurat, Butylene Glykol Caprylate/ Caprate
- Fettsäureglyceride, bevorzugt Monoglyceride oder Diglyceride
- Lösungsvermittler, bevorzugt aus der Gruppe Dimethyl Isosorbide oder Diethylene glycol monoethyl ether, PEG-Ester, Peceol (Glycerolmonooleat mit einem Monoester-Gehalt von 32 - 52 %), Labrasol (polyoxyethylierte Caprylcaprinsäureglyceride), Akoline MCM (Caprylic/ Capric Glycerides, PEG-40-Hydrogenated Castor Oil, Plurol Oleique (Polyglyceryl-6 Dioleate), Gelicire 44/14

Auch beliebige Abmischungen solcher Öl- und Wachskomponenten sind vorteilhaft im Sinne der vorliegenden Erfindung einzusetzen.

Um das Hautgefühl der Emulsionen zu verbessern, kann die Ölphase ferner einen Gehalt an cyclischen oder linearen Silikonölen aufweisen. Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Die wäßrige Phase der Formulierungen im Sinne der vorliegenden Erfindung enthält gegebenenfalls vorteilhaft
- Alkohole, Diole oder Polyole niedriger C-Zahl, sowie deren Ether, vorzugsweise Ethanol, Isopropanol, Propylenglykol, Methylpropandiol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Xanthangummi und/oder Hydroxypropylmethylcellulose, jeweils einzeln oder in Kombination.

Es ist auch vorteilhaft im Sinne der vorliegenden Erfindung, kosmetische Zubereitungen zu erstellen, die einen Gehalt an UV-Schutzsubstanzen enthalten. So werden z. B. in Tagescremes oder Hautlotions gewöhnlich UV-A- bzw. UV-B-Filtersubstanzen eingearbeitet. Darüber hinaus stellen UV-Schutzsubstanzen, ebenso wie Antioxidantien und gegebenenfalls Konservierungsstoffe, einen wirksamen Schutz gegen den Verderb der Zubereitungen selbst und UVempfindlicher Inhaltsstoffe dar. So können die UV-Filtersubstanzen im Sinne der vorliegenden Erfindung beispielsweise dazu dienen, die Stabilität des Silymarins zu verbessern bzw. das Silymarin vor UV-induzierter Zersetzung zu schützen.

Dementsprechend enthalten die Zubereitungen im Sinne der vorliegenden Erfindung vorzugsweise mindestens eine übliche UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase dispergiert vorliegen können.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden.

Solche Pigmente können im Sinne der vorliegenden Erfindung vorteilhaft oberflächlich behandelt ("gecoatet") sein, wobei beispielsweise ein amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophoben Schicht versehen werden.

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Methylene Bis-Benztriazolyl Tetramethylbutylphenol], welches unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 verkauft wird.

Weitere vorteilhafte UV-A-Filtersubstanzen sind die Phenylen-1,4-bis-(2-benzimidazyl)-3,3'-5,5'-tetrasulfonsäure und ihre Salze, insbesondere das Phenylen-1,4-bis-(2-benzimidazyl)-3,3'-5,5'-tetrasulfonsäure-bis-natriumsalz mit der INCI-Bezeichnung Bisimidazylate, welches beispielsweise unter der Handelsbezeichnung Neo Heliopan AP bei Symrise erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird.

Ferner vorteilhaft sind auch die UV-A-Filter 2-(4'-Diethylamino-2'-hydroxybenzoyl)-benzoesäurehexylester (auch: Aminobenzophenon), der unter dem Namen Uvinul® A Plus bei der Fa. BASF erhältlich ist, und Uvasorb® K2A (2,4-bis-[5-1 (dimethylpropyl)benzoxazol-2-yl-(4-phenyl)-imino]-6-(2-ethylhexyl)-imino-1,3,5-triazin, CAS Nr. 288254-16-0), der bei 3V Sigma erhältlich ist, welche einzeln oder in Mischungen eingesetzt werden können. Besonders vorteilhaft ist es, 2-(4'-Diethylamino-2'-hydroxybenzoyl)-benzoesäurehexylester und 4-(tert.-Butyl)-4'-methoxydibenzoylmethan in den erfindungsgemäßen Formulierungen zu kombinieren, insbesondere wenn ein hoher UV-A-Schutz gewünscht ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCI: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird;

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z.B. unter der Handelsbezeichnung Tinosorb® M bei der CIBA-Chemikalien GmbH erhältlich ist und unter bestimmten Bedingungen auch in gelöster Form vorliegen kann.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)-ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter, wie z. B Polysilicone-15, das auch unter dem Handelsnamen Parsol SLX erhältlich ist.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Es ist bevorzugt im Sinne der vorliegenden Erfindung Polysilicone-15 und 2-Phenylbenzimidazol-5-sulfonsäure zu kombinieren.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A- und/oder UV-B-Filter in kosmetische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

| **Beispiel 1** | **Gew.-%** |
|---|---|
| Pflegecreme | |
| Glycerylstearat | 2 |
| PEG-40-Stearat | 0.8 |
| Stearylalkohol | 1 |
| Cetylalkohol | 1 |
| Hydrierte Kokosfettglyceride (Hydrogenated Coco Glycerides) | 2 |
| Propylene Glykol Isostearate | 2 |
| C12-C15 Alkylbenzoate | 2 |
| Cyclometicone | 3 |
| Dicaprylylether | 2 |
| Tocopherylacetat | 1 |
| Natriumascorbylphosphat | 0,1 |
| Silibinin | 0,1 |
| p-Hydroxybenzoesäurealkylester | 0,2 |
| Methylpropanediol | 4 |
| Aristoflex AVC (Ammonium Polyacryldimethyltauramid/Vinylformamidcopolymer) | 0,3 |
| Allantoin | 0.1 |
| Licochalkone | 0.025 |
| Pigmente | 0.5 |
| Lecithin | 0.1 |
| EDTA | 0,2 |
| Glycerin | 8 |
| wasser- oder öllösliche Farbstoffe | 0,05 |
| Tapiokastärke | 2 |
| Parfüm | q.s. |
| Wasser | ad 100 |

| **Beispiel 2** | **Gew.-%** |
|---|---|
| O/W-Tagescreme | |
| Glyceryl stearate Citrate | 2 |
| Cetylalkohol | 2 |
| Myristyl Alcohol | 2 |
| C₁₂₋₁₅ Alkylbenzoat | 2 |
| Alkyllaktate | 2 |
| Dimethicon | 1 |
| Dicarprylylcarbonat | 3 |
| Ethylhexylcyanodiphenylacrylat (Octocrylen) | 5 |
| Butylmethoxydibenzoylmethan | 2 |
| Citronensäure, Natriumsalz | 0.1 |
| Silybum Marianum Extrakt | 0,1 |
| Phenoxyethanol | 0,4 |
| p-Hydroxybenzoesäurealkylester (Paraben) | 0,2 |
| Alpha Glucosyl Rutin | 0.03 |
| EDTA | 0,2 |
| Ethanol (vergällt) | 2 |
| Ammoniumacryloyldimethyltaurate/ Vinylpyrrolidoncopolymere | 0,5 |
| Glycerin | 10 |
| Q 10 | 0.1 |
| Parfüm | q.s. |
| Wasser | ad 100 |

| **Beispiel 4** | **Gew.-%** |
|---|---|
| O/W Creme | |
| Polyglyceryl-3-Methylglucosedistearat | 3 |
| Cetylalkohol | 3 |
| C₁₂₋₁₅ Alkylbenzoat | 2 |
| Butylenglycol Dicaprylat/Dicaprat | 2 |
| Caprylsäure/Caprinsäuretriglyceride | 2 |
| Hydriertes Polydecen | 1 |
| Dimethylpolysiloxan (Dimethicon) | 1 |
| Isodecylneopentanoat | 4 |
| Bis-Ethylhexyloxyphenolmethoxyphenyltriazin | 1 |
| Ethylhexylmethoxycinnamat | 5 |
| Siliphos | 0,2 |
| Natriumascorbylphosphat | 0,1 |
| EDTA | 0,2 |
| Phenoxyethanol | 0,4 |
| Iodopropinylbutylcarbamat | 0,05 |
| p-Hydroxybenzoesäurealkylester (Paraben) | 0,4 |
| Carbomer 980 | 0,1 |
| Hydroxypropylstärkephosphatester | 0,2 |
| Glycerin | 5 |
| Additive (Distärkephosphat, Talkum, BHT) | 0,2 |
| Parfüm | q.s. |
| Wasser | ad 100 |

| **Beispiel 5** | **Gew.-%** |
|---|---|
| **Hydrodispersion** | |
| Silibinin | 0,05 |
| Lecithin | 0.1 |
| Aloe vera | 0.1 |
| Acrylates/C10-30 Alkyl Acrylate Crosspolymer | 0.1 |
| EDTA | 0,2 |
| Polyvinylpyrrolidon-Hexadecen Copolymer | 0,4 |
| Paraben | q.s. |
| Ethylhexylglycerin | 0,4 |
| Xanthan Gum | 0,15 |
| Carrageenan | 0,15 |
| Cyclomethicone | 2 |
| Ethylhexyl Methoxycinnamate | 5 |
| Phenylbenzimidazole Sulfonic Acid | 1 |
| Glycerin | 6 |
| Butylenglycol | 2 |
| BHT | 0.05 |
| Parfüm | q.s. |
| Wasser | ad 100 |

## Patentansprüche

1. Verwendung von kosmetischen Formulierungen mit einem Gehalt an Silybin und/oder Silydianin und/oder Silychristin oder Silymarin zur Erhöhung bzw. Wiederherstellung der Barriereeigenschaften der Haut.

2. Verwendung nach Anspruch 1, **dadurch gekennzeichnet, dass** die kosmetische Formulierung - bezogen auf das Gesamtgewicht der Formulierung - 0,001 Gew.-% bis 10 Gew.-% an Silymarin enhält.

3. Verwendung nach einem der vorhergehenden Ansprüche **dadurch gekennzeichnet, dass** die kosmetische Formulierung - bezogen auf das Gesamtgewicht der Formulierung - 0,01 bis 5 Gew.-% an Silymarin enhält.

4. Verwendung nach einem der vorhergehenden Ansprüche **dadurch gekennzeichnet, dass** die kosmetische Formulierung - bezogen auf das Gesamtgewicht der Formulierung - 0,02 bis 0,5 Gew.-% an Silymarin enhält.

5. Verwendung nach einem der vorhergehenden Ansprüche zur Stärkung der mechanischen Stabiliät der Haut.

6. Verwendung nach einem der vorhergehenden Ansprüche zur Erhöhung der Spannkraft der Haut.

7. Verwendung nach einem der vorhergehenden Ansprüche zum Schutz der Haut vor Austrocknung.

8. Verwendung nach einem der vorhergehenden Ansprüche zum Schutz der Haut vor Umwelteinflüssen.

9. Verwendung nach einem der vorhergehenden Ansprüche zum Schutz der Haut von Kindern und Jugendlichen.
